Europäisches Patentamt

European Patent Office

Office européen des brevets

(19)

(11) EP 0 830 089 B1

(12) EUROPÄISCHE PATENTSCHRIFT

(45) Veröffentlichungstag und Bekanntmachung des
Hinweises auf die Patenterteilung:
**08.09.2004 Patentblatt 2004/37**

(21) Anmeldenummer: **96921947.6**

(22) Anmeldetag: **07.06.1996**

(51) Int Cl.7: **A61B 18/20**

(86) Internationale Anmeldenummer:
**PCT/EP1996/002486**

(87) Internationale Veröffentlichungsnummer:
**WO 1996/041577 (27.12.1996 Gazette 1996/56)**

(54) **GEPULSTE LICHTQUELLE ZUM ABTRAGEN VON BIOLOGISCHEM GEWEBE**

PULSED LIGHT SOURCE FOR CUTTING AWAY BIOLOGICAL TISSUE

SOURCE DE LUMIERE PULSEE D'ABLATION DE TISSUS BIOLOGIQUES

(84) Benannte Vertragsstaaten:
**AT BE CH DE ES FR GB IT LI SE**

(30) Priorität: **08.06.1995 DE 19521003**

(43) Veröffentlichungstag der Anmeldung:
**25.03.1998 Patentblatt 1998/13**

(73) Patentinhaber: **Carl Baasel Lasertechnik GmbH
D-82319 Starnberg (DE)**

(72) Erfinder: **HIBST, Raimund
D-89155 Erbach (DE)**

(74) Vertreter: **Feldkamp, Rainer, Dipl.-Ing.
Garmischer Strasse 4
80339 München (DE)**

(56) Entgegenhaltungen:
**EP-A- 0 164 751**      **EP-A- 0 657 141**
**WO-A-92/18057**      **DE-A- 3 233 671**
**DE-A- 3 841 503**      **DE-A- 3 904 287**
**DE-A- 3 934 646**      **DE-A- 4 015 066**

**Beschreibung**

Die Erfindung bezieht sich auf eine gepulste Lichtquelle zum Abtragen von biologischem Gewebe.

Es ist aus vielen medizinischen Anwendungen bekannt, daß mit Hilfe von hinreichend intensivem Licht, insbesondere Laserstrahlung, Gewebe abgetragen oder geschnitten werden kann. Mit dem Abtrag ist eine Erwärmung des umliegenden Gewebes verbunden. Das Ausmaß dieser Erwärmung ist insbesondere durch die Wellenlänge der verwendeten Strahlung bzw. den hiervon abhängigen Absorptionskoeffizienten des Gewebes und die Bestrahlungsstärke bestimmt. Bei hoher Absorption im Gewebe und niedriger Bestrahlungsstärke, wie es z.B. beim $CO_2$-Dauerstrichlaser der Fall ist, wird Gewebe auf pyrolytischem Wege mit relativ großer Wärmenebenwirkung verdampft. Im typischen Fall ist der in Weichgewebe gebildete Krater oder Schnitt von einer Karbonisationsschicht, einer durch Vakuolen aufgelockerten Zone, einer Koagulationszone und einem reversibel thermisch geschädigten Bereich umgeben. Die durch die Erwärmung erzeugte Koagulation des Gewebes und die damit verbundene Hämostase ist in vielen Fällen von praktischem Vorteil, weil sie nicht blutende Schnitte ermöglicht. Für Anwendungen hingegen, bei denen es auf eine möglichst geringe Schädigung des verbleibenden Gewebes und einen guten Wundheilungsverlauf ankommt, sind große thermische Wirkungen nachteilig. Ungünstig ist ebenfalls eine Karbonisation der Gewebeoberfläche, wie sie beim Schneiden mit Dauerstrichlasern auftritt. Es wurde bereits versucht, bei derartigen Lasern durch eine Heraufsetzung der Bestrahlungsstärke bei gleichzeitiger Verkürzung der Einwirkzeit die thermischen Schäden zu verringern.

Auf der anderen Seite haben Forschungen der letzten Jahre gezeigt, daß mit gepulsten Lichtquellen hoher Leistung und einer im Ultravioletten oder Infraroten angesiedelten Wellenlänge, z.B. TEA-$CO_2$-, Er:YAG-, Er:YSGG- oder Excimer-Lasern, Hartoder Weichgewebe ohne Karbonisation durch einen sehr effektiven thermomechanischen Ablationsprozeß mit nur geringen thermischen Schäden abgetragen werden kann. So beträgt bei Weichgewebe der nach Einsatz des freilaufenden Er:YAG-Lasers koagulierte Randsaum in vivo nur etwa 30-40 µm. Dies ist für die Behandlung oberflächlicher Hautläsionen oder für die kosmetische Chirurgie von besonderem Interesse, weil eine über den Abtrag hinausgehende Schädigung des Gewebes weitgehend vermieden wird. Wird jedoch die Kapillarschicht des Gewebes erreicht, kommt durch austretendes Blut der Abtrag zum Erliegen.

Bei allen bisher verwendeten chirurgischen Anwendungen von Lichtquellen sind die Abtragseigenschaften und die Wärmenebenwirkungen gekoppelt, in der Weise, daß ein präziser Abtrag mit hoher Abtragseffizienz immer mit einer geringen Wärmenebenwirkung verbunden ist und umgekehrt. Eine bekannte Möglichkeit, verschiedene Wärmenebenwirkungen zu erreichen, liegt in der Kombination mehrer Lichtquellen verschiedener Wellenlänge in einem Gerät. Der für ein gleichzeitiges Schneiden und Koagulieren erforderliche Parallelbetrieb beider Lichtquellen bedingt allerdings einen hohen apparativen Aufwand.

Aus der DE 39 34 646 A1 ist ein Verfahren bzw. eine Vorrichtung der eingangs genannten Art bekannt, bei dem bzw. bei der eine gezielte Verdampfung ohne partielle Zersetzung oder Verbrennung dadurch erreicht werden soll, daß die beim Pyrolyseprozeß entstehende Leuchterscheinung als Regelsignal für die Steuereinheit verwendet wird. Die Steuereinheit wird hierdurch so gesteuert, daß entweder die Laserleistung, das Taktverhältnis oder die Pulsenergie verändert werden.

Weiterhin ist aus der DE 32 33 671 A1 eine Laservorrichtung mit einer Speichereinrichtung zum Speichern einer Vielzahl von Datensätzen bekannt, die Parameter für die Arbeitsbedingungen für eine bestimmte Laserstrahlung angeben. Einzelheiten über die einzelnen Parameter und damit eine optimale Abtragung von Gewebe sind hier jedoch nicht angegeben.

Der Erfindung liegt die Aufgabe zugrunde, eine gepulste Lichtquelle der eingangs genannten Art zu schaffen, die es ermöglicht, mit nur einer einzigen Wellenlänge Gewebe gleichzeitig präzise und mit geringen thermischen Nebenwirkungen sowie ohne Karbonisierung der Oberfläche abzutragen und, unabhängig vom Abtrag, gezielt und steuerbar zu erwärmen, um z.B. eine für den Einsatzzweck spezifische Koagulationszone zu erzeugen.

Diese Aufgabe wird durch die im Anspruch 1 und Anspruch 2 angegebenen Merkmale gelöst.

Vorteilhafte Ausgestaltungen und Weiterbildungen der Erfindung ergeben sich aus den Unteransprüchen.

Mit der erfindungsgemäßen gepulsten Lichtquelle kann biologisches Gewebe präzise und mit geringen thermischen Nebenwirkungen abgetragen und zusätzlich in variablem Ausmaß erwärmt werden.

Die Lichtemission der erfindungsgemäßen Lichtquelle ist steuerbar derart moduliert, daß innerhalb eines Impulszyklus auf einen zum Gewebeabtrag verwendeten Strahlungsimpuls hoher Leistung in definierter Zeit eine Lichtstrahlung mit verringerter Bestrahlungsstärke folgt, die die Form eines eine verringe Leistung aufweisenden Endabschnittes des Strahlungsimpulses oder die Form einer Impulsserie aus einem oder mehreren Strahlungsimpulsen haben kann, deren Leistung oder Energieinhalt zu einem Gewebeabtrag nicht ausreichend ist und damit nur zur Erwärmung des Gewebes führt.

Die erfindungsgemäße gepulste Lichtquelle erlaubt eine bislang ungekannte Anwendungsbreite einer einzelnen Strahlungsquelle für chirurgische Applikationen bei geringem apparativem Aufwand, die von der Präzisionschirurgie mit geringen thermischen Schäden in nicht oder nur schwach durchbluteten Arealen bis zum Entfernen von durchblutetem Gewebe mit Hämostase reicht. Da die Wärmenebenwirkung und damit die Dicke der Koagulationszone dem individuellen Eingriff angepaßt werden kann, läßt sich in jedem Fall eine gerade ausreichende und mithin minimal schädigende thermische Nekrosezone erzeugen. Außerdem führt eine derartig vergrößerte Koagulationszone nicht zu Einbußen in der Schnittqualität.

Die erfindungsgemäße Lichtquelle ist vorzugsweise eine Ultraviolett- oder Infrarot-Lichtquelle.

Der erste Strahlungsimpuls des Impulszyklus entspricht der Strahlungsemission, wie sie gängigerweise zur schädigungsarmen Ablation von biologischem Gewebe eingesetzt wird. Der Gewebeabtrag setzt ein, wenn an seiner Oberfläche eine bestimmte, vom Gewebetyp und von der Bestrahlungsstärke abhängige Energie pro Volumenelement $H_{abl}$ akkumuliert ist. Dies entspricht einem, ebenfalls von dem Gewebe und den Bestrahlungsparametern abhängigen Schwellenwert der Bestrahlung ($F_s$). Ein Teil der eingestrahlten Energie verbleibt am Ende des Impulses im Gewebe, erwärmt den Randbereich der Krater oder Schnitte und führt zu den beschriebenen thermischen Nebenwirkungen, insbesondere der Koagulation des Gewebes.

Gemäß einer bevorzugten Ausgestaltung der Erfindung werden die Parameter des ersten Strahlungsimpulses der Impulsserie so gewählt, daß die im Zusammenhang mit dem Gewebeabtrag entstehende Erwärmung und Gewebebeschädigung klein ist. Dies wird durch die Kombination aus hoher Bestrahlungsstärke und hoher Absorption im Gewebe erreicht (typischer Absorptionskoeffizient größer 10 cm$^{-1}$).

Die hierzu verwendete Lichtquelle ist vorzugsweise ein gepulster Er:YAG-, Er:YSGG-, Ho:YAG-, Tm:YAG-, CO-, $CO_2$- oder Excimer-Laser.

Einige Daten hierzu sind der Veröffentlichung von R. Hibst und R. Kaufmann, 'Vergleich verschiedener Mittelinfrarot-Laser für die Ablation der Haut', Lasermedizin Vol. 11 (1995), S. 19-26, zu entnehmen.

Typische Werte für den Er:YAG-Laser sind:

· für den Abtrag pro Volumenelement erforderliche Energie $H_{abl}$ = 1, 5 kJcm$^{-3}$

· Schwellenwert der Bestrahlungsstärke etwa 1 Jcm$^{-2}$

· Impulsdauer im Bereich von 150 bis 600 µs

· Bestrahlung im klinischen Einsatz an der Haut etwa 5 - 20 Jcm$^{-2}$

· mittlere Bestrahlungssärke einige 10 kWcm$^{-2}$

· Fleckgröße bei flächenhaftem Abtrag 1 bis 3 mm im Durchmesser

Die laserbezogene Leistung berechnet sich aus der Bestrahlungsstärke und der Fleckgröße.

Die durch den abtragenden Impuls verursachte Koagulationszone wird erfindungsgemäß dadurch vergrößert, daß anschließend an den zum Abtrag führenden kurzen Impuls eine jeweils nachfolgende Lichtstrahlung mit einer Bestrahlungsstärke und/oder Bestrahlung emittiert wird, die für das Abtragen von Gewebe nicht ausreicht, jedoch eine Wärmewirkung ergibt.

Diese nachfolgende Lichtstrahlung in Form eines Impulsendabschnittes verringerter Bestrahlungsstärke oder von mindestens einem, vorzugsweise jedoch mehreren Lichtimpulsen ist hinsichtihrer Leistung oder Energie so bemessen, daß bei einer vorgegebenen Größe des Bestrahlungsfeldes der Abtragschwellenwert des Gewebes nicht erreicht wird.

Gemäß einer Ausgestaltung der Erfindung wird hierzu mindestens ein Impuls mit geringer Bestrahlungsstärke verwendet. Damit das Gewebe nicht abgetragen und nur aufzuheizt wird, werden Impulse mit einer so geringen Bestrahlungsstärke verwendet, daß infolge der Wärmeleitung die an der Oberfläche akkumulierte Energie pro Volumentelement unter $H_{abl}$ bleibt, d. h. es wird der für den Abtrag erforderliche Schwellenwert der Bestrahlungsstärke unterschritten.

Zur Abschätzung der Obergrenze der Bestrahlungsstärke kann angenommen werden, daß sich die pro Volumenelement einstellende Energie H aus der durch Lichtabsorption gegebenen Energiezufuhr und einem zu H proportionalen Energieverlust ergibt:

$$\frac{dH}{dt} = \mu \cdot I_0 - \frac{1}{\tau} \cdot H$$

($I_0$: Bestrahlungsstärke, ρ: Absorptionskoeffizient). Die als Proportionalitätsfaktor für die Verlustrate eingesetzte thermische Relaxationszeit τ läßt sich aus bekannten Formeln abschätzen. Sie nimmt quadratisch mit dem erwärmten Volumen, und daher mit ansteigendem ρ, ab. Der Schwellenwert der Bestrahlungsstärke $I_s$ ist dann erreicht, wenn im Gleichgewicht (dH/dt = 0) die Energiedichte an der Oberfläche gleich $H_{abl}$ ist. Damit ergibt sich aus obiger Gleichung:

$$I_s = \frac{H_{abl}}{\mu \cdot \tau}$$

Für den Er:YAG-Laser läßt sich für den Anfang der Bestrahlung die thermische Relaxationszeit des Gewebes zu einigen µs abschätzen, so daß sich mit den übrigen Werten (s.o.) eine Bestrahlungsstärke $I_s$ im kWcm$^{-2}$-Bereich errechnet. Mit zunehmender Vergrößerung des erwärmten Bereiches nimmt $I_s$ dann ab. Der genaue Verlauf ist hier schwer zu berechnen. Für eine Schicht von beispielsweise 80 µm Dicke ist die thermische Relaxationszeit etwa 30 ms, was zu einer maximal zulässigen Bestrahlungsstärke von etwa 5 Wcm$^{-2}$ führt. Eine vorteilhafte Ausgestaltung dieser Alternative ist daher ein Verlauf mit abnehmender Bestrahlungsstärke. Die Bestrahlungsstärke (Leistung) und die Dauer des Impulses bestimmt dann das Ausmaß der Erwärmung.

Wenn der erforderliche Unterschied in der Bestrahlungsstärke zwischen den abtragenden und den erwärmenden Impulsen bei einem vorgegeben Laser technisch schwer zu realisieren ist, so ist gemäß einer weiteren Ausgestaltung der Erfindung vorgesehen, eine Folge von Impulsen mit einem Energieinhalt unter dem Abtragschwellenwert zu verwenden.

Als Anhaltspunkt für diesen Schwellenwert können die aus den Abtragmessungen bestimmten Schwellenwerte $F_s$ (s.o.) benutzt werden. Die Schwellenwerte vergrößern sich mit abnehmender Bestrahlungsstärke (sie werden theoretisch unendlich bei einer Bestrahlungsstärke von $I_s$) und erniedrigen sich bei vorgewärmtem Gewebe. Für den Er:YAG-Laser würde man also zunächst von $F_s$ = 1Jcm$^{-2}$ ausgehen und im Experiment die Bestrahlungsstärke jedes einzelnen Impulses oder seine Dauer so verändern, daß gerade kein Abtrag mehr stattfindet. Die Einzelfaktoren Bestrahlungsstärke und Impulsdauer richten sich nach den technischen Erfordernissen der Lichtquelle, für den Effekt entscheidend ist in erster Linie ihr Produkt.

Gemäß einer Ausführungsform können die Bestrahlungsstärke und die Dauer der auf den ersten Strahlungsimpuls folgenden Impulse voneinander verschieden sein. Dies bietet sich z.B. für den Er:YAG-Laser an, wenn zur Versorgung der Pump-Blitzlampe dieses Lasers die Energie einer einzigen Kondensatorbank für die Erzeugung der ganzen Impulsserie genutzt wird. Durch die abnehmende Spannung werden die Laserimpulse zunehmend schwächer, was aber durch eine entsprechend verlängerte Impulsdauer ausgeglichen werden kann.

Der optimale zeitliche Abstand zwischen den Unterimpulsen bzw. zwischen dem abtragenden Impuls und der Impulsserie für das Heizen ergibt sich aus der thermischen Relaxationszeit der Gewebeoberfläche. Um möglichst viel Energie in das Gewebe einbringen zu können ohne es abzutragen, ist es günstig, zwischen zwei derartigen Impulsen die Gewebeoberfläche gegenüber der zu einem Abtrag führenden Temperatur abkühlen zu lassen. Um gleichzeitig eine große Tiefe der Koagulation zu erzeugen, sollte dieses Abkühlen nicht bis zur (physiologischen) Ausgangstemperatur (typisch 37°C) erfolgen. Vielmehr sollte die Nachheizung durch den folgenden Impuls spätestens dann erfolgen, wenn die Oberfläche die zur angestrebten Koagulation erforderliche Temperatur von etwa 60°C bis 70°C erreicht hat. Diese Zeit nimmt mit der optischen Penetrationstiefe der verwendeten Strahlung zu.

Außerdem hängt das Abkühlverhalten der Oberfläche von ihrer Vorgeschichte ab. Beim ersten Laserpuls führt die oberflächliche Erwärmung des Gewebes zu einem sehr steilen Temperaturgradienten mit einem, bedingt durch die Wärmeleitung, entsprechend schnellem Abklingen der Temperatur. Durch die Wärmeleitung werden auch Gewebeschichten unter der Oberfläche aufgeheizt, so daß der Temperaturgradient für einen nachfolgenden Heizpuls kleiner ist. Die Zunahme der thermischen Relaxationszeit mit der Zahl der Heizpulse ist aus einer Messung der Oberflächentemperatur erkennbar. Eine optimierte Folge von Heizpulsen wird deshalb im allgemeinen unterschiedliche zeitliche Abstände zwischen den einzelnen Pulsen aufweisen. Analog wird der Energieinhalt der Einzelpulse unterschiedlich sein.

Modellrechnungen sowie Messungen zeigen für den Er:YAG-Laser, daß die für eine Koagulation der Haut in vivo erforderliche Temperaturerhöhung von 30 K bis 40K einige ms nach dem Impulsende wieder an der Oberfläche erreicht ist. Für den Ho:YAG-Laser ist etwa mit dem 20-fachen dieses Wertes zu rechnen. Die genauen Zeiten sind in jedem Fall für das betrachtete Gewebe und die verwendete Wellenlänge experimentell zu bestimmen.

Für andere Effekte als die Koagulation, z.B. zur Hyperthermie, sind selbstverständlich andere Temperaturen und Zeiten maßgeblich, die der Fachmann ohne weiteres ermitteln kann.

Bei dieser Ausführungsform der zum Erwärmen verwendeten Impulsserie kann die insgesamt (pro Oberflächenelement) in das Gewebe eingebrachte Energie und damit die Koagulationstiefe vorteilhaft durch die Anzahl der auf den ersten Impuls folgenden Impulse in der Impulsserie gesteuert werden.

Bei einer abgeänderten Ausführungsform kann als Alternative zur Vorgabe fester Parameter für die einzelnen Heizimpulse eine Steuerung der Pulsenergien, -dauern und Pausen auf der Basis der kontiuierlich oder intermittierend gemessenen Oberflächentemperatur verwendet werden. Sobald die Oberflächentemperatur einen vorgegebenen Minimalwert (z.B. 70°C) unterschreitet, wird der Laser aktiviert. Die Laseremission wird wieder gestoppt, wenn der voreingestellte obere Grenzwert (z.B. 200°C) erreicht wird.

Bei dieser Realisation der zum Erwärmen verwendeten Pulsserie kann die insgesamt (pro Oberflächenelement) in das Gewebe eingebrachte Energie und damit die Koagulationstiefe vorteilhaft durch die Anzahl der auf den ersten Impuls folgenden Impulse in der Pulsserie gesteuert werden.

Selbstverständlich kann die optimierte Serie von Heizpulsen auch ohne den abtragenden Puls zum Koagulieren eingesetzt werden. Ebenso kann es vorteilhaft sein, die Heizpulse (auch) vor dem abtragenden Puls zu applizieren, wenn z.B. infiziertes Gewebe vor dem Abtrag, der mit einem Versprengen von Gewebefragmenten verbunden ist, abgetötet werden soll.

Die Erfindung wird nachfolgend anhand der Zeichnungen noch näher erläutert.

In den Zeichnungen zeigen:

Fig. 1     einen Schnitt durch einen Gewebebereich nach Bestrahlung bei hoher Gewebeabsorption und niedriger Bestrahlungsstärke,

Fig. 2     einen Schnitt durch einen Gewebebereich nach Bestrahlung bei hoher Gewebeabsorption und hoher Bestrahlungsstärke,

Fig. 3 einen Schnitt durch einen Gewebebereich nach Bestrahlung mit einer gepulsten Lichtquelle gemäß der Erfindung,

Fig. 4 eine erste Ausführungsform eines Impulses, der einen eine Abtragung hervorrufenden Impulsanfangsabschnitt und einem hierauf folgenden Impulsendabschnitt mit verringerter Bestrahlungstärke,

Fig. 5 eine bevorzugte Ausführungsform einer Impulsfolge mit einem ersten, eine Abtragung hervorrufenden Impuls und einem hierauf folgenden Impuls mit abnehmender Bestrahlungstärke,

Fig. 6 eine weitere Ausführungsform einer Impulsfolge mit einem ersten, eine Abtragung hervorrufenden Impuls und einer hierauf folgenden Serie von Impulsen mit zunehmend schwächeren, jedoch entsprechend verlängerten Impulsen,

Fig. 7 eine Ausführungs einer Vorrichtung mit einer Steuerung des Lasers in Abhängigkeit von der Oberflächentemperatur des Gewebes.

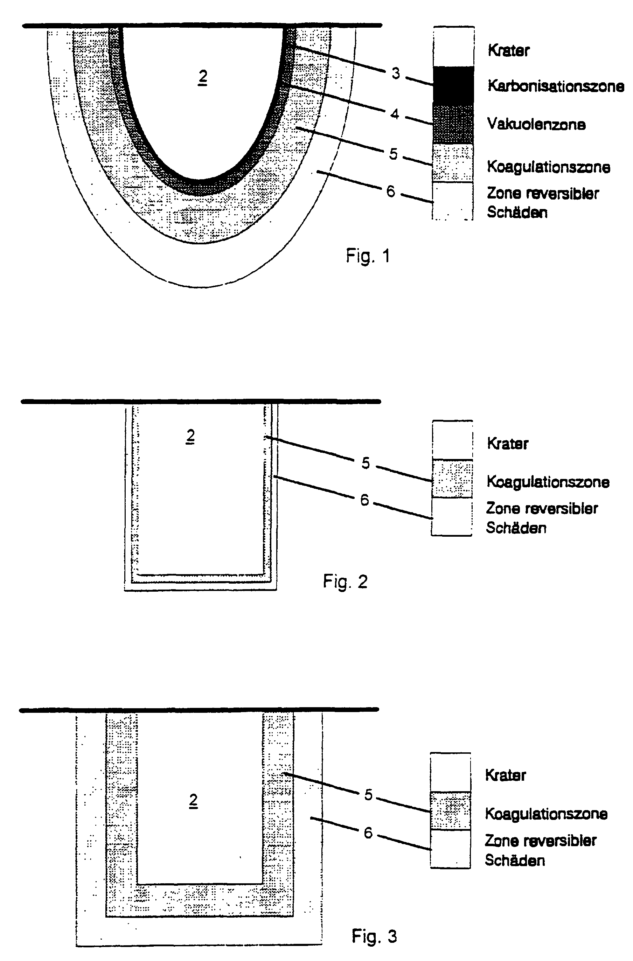

In Fig. 1 ist ein Schnitt durch einen Gewebebereich gezeigt, wie er sich beispielsweise bei hoher Absorption im Gewebe und niedriger Bestrahlungsstärke ergibt. Dies ist z.B. beim $CO_2$-Dauerstrichlaser der Fall, der auf die Gewebeoberfläche 1 gerichtet wird. Der im Gewebe gebildete Krater oder Schnitt 2 ist von einer Karbonisationszone 3, einer durch Vakuolen aufgelockerten Zone 4, einer Koagulationszone 5 und einer reversibel thermisch geschädigten Zone 6 umgeben. Die durch die Erwärmung erzeugte Koagulation des Gewebes und die damit verbundene Hämostase ist in vielen Fällen von praktischem Vorteil, weil sie nicht blutende Schnitte ermöglicht. Für Anwendungen, bei denen es auf eine möglichst geringe Schädigung des verbleibenden Gewebes und einen guten Wundheilungsverlauf ankommt, sind große thermische Wirkungen nachteilig. Ungünstig ist auch die Karbonisation der Gewebeoberfläche.

In Fig. 2 ist ein der Fig. 1 entsprechender Schnitt gezeigt, der die Bestrahlung mit einer gepulsten Lichtquelle hoher Leistung und einer im Ultravioletten oder Infraroten angesiedelten Wellenlänge zeigt. Beispiele für eine derartige Lichtquelle sind TEA-$CO_2$-, Er: YAG-, Er:YSGG- oder Excimer-Laser. Hierbei wird Hart- oder Weichgewebe ohne Karbonisation durch einen sehr effektiven thermomechanischen Ablationsprozeß mit nur geringen thermischen Schäden abgetragen. Die bei Weichgewebe nach Einsatz des freilaufenden Er: YAG-Lasers koagulierte Zone 5 hat in vivo nur eine Stärke von etwa 30-40 μm. Dies ist für die Behandlung oberflächlicher Hautläsionen oder für die kosmetische Chirurgie von besonderem Interesse, weil eine über den Abtrag hinausgehende Schädigung des Gewebes weitgehend vermieden wird. Wird jedoch die Kapillarschicht erreicht, kommt durch austretendes Blut der Abtrag zum Erliegen.

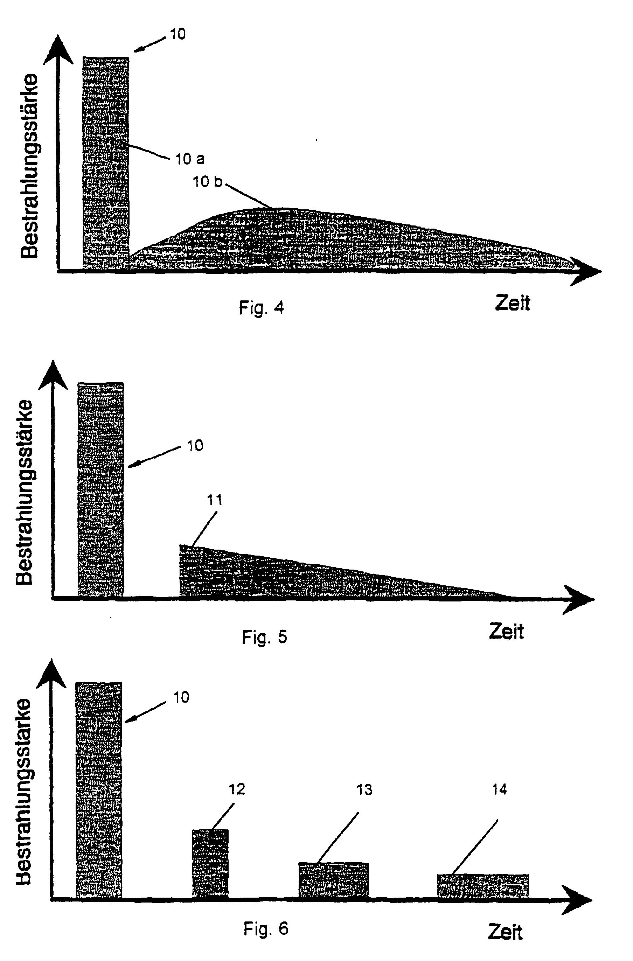

Fig. 3 zeigt einen den Fig. 1 und 2 entsprechenden Schnitt durch ein Gewebe nach Bestrahlung mit der erfindungsgemäßen gepulsten Lichtquelle. Wie dies anhand der Fig. 4 und 5 nachfolgend noch näher erläutert wird, wird hierbei die Lichtemission einer gepulsten Ultraviolett- oder Infrarot-Lichtquelle steuerbar derart moduliert, daß innerhalb eines Impulszyklus auf einen zum Gewebeabtrag ausreichenden Impuls hoher Leistung in definierter Zeit eine Impulsserie aus einem oder mehrer Pulsen folgt, deren Leistung oder Energieinhalt zu einem Gewebeabtrag nicht ausreichend ist und damit nur zur Erwärmung des Gewebes führt. Hierbei ist der Krater 2 von einer Koagulationszone 5 mit steuerbarer Größe umgeben. Auf diese Weise ist es möglich, Gewebe gleichzeitig präzise, mit geringen thermischen Nebenwirkungen und ohne Karbonisierung der Oberfläche abzutragen und, unabhängig vom Abtrag, gezielt und steuerbar zu erwärmen.

Fig. 4 zeigt eine erste Ausführungsform eines Impulses zur Erzielung der in Fig. 3 gezeigten Abtragung. Hierbei umfaßt jeder Impuls einen kurzen, ersten, zur Abtragung ausreichenden, Impulsanfangsabschnitt 10 und einen nachfolgenden Impulsendabschnitt mit verringerter Bestrahlungsstärke. Hinsichtlich der einzelnen Parameter der Impulsabschnitte wird auf die vorstehende Diskussion verwiesen.

Fig. 5 zeigt eine zweite Ausführungsform einer Impulsfolge zur Erzielung der in Fig. 3 gezeigten Abtragung. Hierbei folgt in einem Impulszyklus auf einen ersten, kurzen, zur Abtragung ausreichenden, Impuls 10 mindestens ein weiterer, von dem Impuls 10 durch einen Zeitabstand getrennter Impuls 11 mit zeitlich abnehmender Bestrahlungsstärke, der lediglich eine Wärmewirkung ergibt.

In Fig. 6 ist eine weitere Ausführungsform einer Impulsfolge gezeigt, bei der in einem Impulszyklus auf den zur Abtragung ausreichenden kurzen Impuls 10 hoher Bestrahlungsstärke eine Folge von Impulsen 12 bis 14 folgt, deren Bestrahlungsstärke jeweils abnimmt, deren Dauer jedoch zunimmt.

Selbstverständlich könnte die Bestrahlungsstärke der auf den ersten Impuls 10 folgenden Impulse 11 bzw. 12 bis 14 und deren Dauer auch konstant sein, solange sie nicht zu einer weiteren Schädigung oder einem Abtrag des Gewebes führen. Weiterhin kann die Anzahl dieser Impulse 12 bis 14 auf der Grundlage der eingangs genannten Kriterien für den jeweiligen Anwendungsfall zweckentsprechend ausgewählt werden.

Eine Alternative zur Vorgabe fester Parameter für die einzelnen Heizimpulse ist die Steuerung der Impulsenergien, -dauern und Pausen auf der Basis der kontinuierlich oder intermittierend, beispielsweise zwischen den einzelnen Impulsen, gemessenen Oberflächentemperatur. Sobald die Oberflächentemperatur einen vorgegebenen Minimalwert (z.B. 70°C) unterschreitet, wird der Laser aktiviert. Die Laseremission wird wieder gestoppt, wenn der voreingestellte obere Grenzwert (z.B. 200°C) erreicht wird.

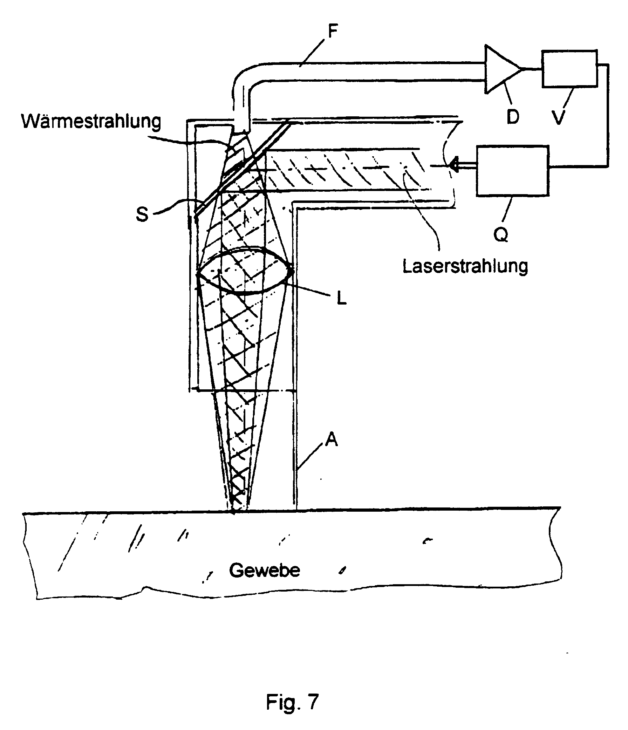

Eine mögliche Ausgestaltung einer derartigen Vorrichtung in Form eines Handstückes ist in Fig. 7 schematisch gezeigt. Die Laserstrahlung von einer Laserquelle Q wird über einen Teilerspiegel S, der für die thermische Strahlung durchlässig ist, umgelenkt und durch eine für Laserstrahlung und Wärmestrahlung transparente Linse L auf das Gewebe fokussiert. Der bestrahlte Oberflächenbereich des Gewebes wird ebenfalls durch die Linse L auf die Endfläche einer die Wärmestrahlung transmittierenden Lichtleitfaser (z.B. eine Silberhalogenidfaser oder Chalcogenidfaser) abgebildet. Diese leitet die Wärmestrahlung zu einem Infrarot-Detektor D. Aus dessen Ausgangssignal, das in einem Verstärken V verstärkt wird, läßt sich nach entsprechender Kalibrierung die Oberflächentemperatur des gerade bearbeiteten Gewebes berechnen, was dann zu der beschriebenen Steuerung des Lasers ausgenutzt werden kann.

Bei dieser Ausführungsform der zum Erwärmen verwendeten Impulsserie kann die insgesamt (pro Oberflächenelement) in das Gewebe eingebrachte Energie und damit die Koagulationstiefe vorteilhaft durch die Anzahl der auf den ersten Impuls folgenden Impulse in der Impulsserie gesteuert werden.

Obwohl vorstehend als Beispiele für die Lichtquelle lediglch Laser-Lichtquellen genannt wurden, sind diese Beispiele in keiner Weise beschränkend, da auch andere Lichtquellen mit entsprechender Wellenlänge und Bestrahlungsstärke, deren Lichterzeugungsprozeß nicht auf dem Laserprinzip beruht, verwendet werden können, wie zum Beispiel gepulste Hochdruck-Gasentladungslampen mit Xenon- oder anderer Gasfüllung.

**Patentansprüche**

1. Gepulste Lichtquelle zum Abtragen von biologischem Gewebe, mit einer Steuereinheit zur Steuerung der Lichtquelle derart, daß diese eine Folge von Impulsen jeweils mit vorgegebener Dauer und Bestrahlungsstärke liefert,
wobei die Steuereinheit derart betreibbar ist, daß die Lichtquelle mit vorgegebener Wiederholfrequenz Impulse (10) gleicher Wellenlänge liefert, wobei die Impulse (10) jeweils einen ersten Impulsabschnitt (10a) kurzer Dauer mit einer zum Abtragen von Gewebe ausreichenden Bestrahlungsstärke und Bestrahlung und einen nachfolgenden Impulsabschnitt (10b) umfassen, dessen Bestrahlungsstärke von dem ersten Impulsabschnitt (10a) getrennt steuerbar ist und für das Abtragen von Gewebe nicht ausreichend ist, jedoch eine Wärmewirkung ergibt.

2. Gepulste Lichtquelle zum Abtragen von biologischem Gewebe, mit einer Steuereinheit zur Steuerung der Lichtquelle derart, daß diese eine Folge von Impulsen jeweils mit vorgegebener Dauer und Bestrahlungsstärke liefert, wobei
die Steuereinheit derart betreibbar ist, daß die Lichtquelle mit vorgegebener Wiederholfrequenz Impulse (10) gleicher Wellenlänge liefert, wobei die Impulse (10) aus mindestens einem ersten Impuls (10) mit einer zum Abtragen von Gewebe ausreichenden Bestrahlungsstärke und Bestrahlung und aus mindestens einem weiteren Impuls (11; 12-14) mit einer von dem mindestens einem ersten Impuls (10) getrennt steuerbaren Bestrahlungsstärke bestehen, welche für das Abtragen von Gewebe nicht ausreichend ist, jedoch eine Wärmewirkung ergibt.

3. Lichtquelle nach einem der Ansprüche 1 oder 2, **dadurch gekennzeichnet, daß** der mindestens eine erste Impuls (10) bzw. erste Impulsabschnitt (10a) eine schädigungsarme Abtragung von Gewebe mit nur geringer Koagulation der Randbereiche der Abtragung ergeben.

4. Lichtquelle nach einem der Anspruch 1 bis 3, **dadurch gekennzeichnet, daß** der mindestens eine erste Impuls (10) bzw. erste Impulsabschnitt (10a) eine hohe Bestrahlungsstärke aufweisen und das emittierte Licht eine hohe Absorption in dem Gewebe aufweist.

5. Lichtquelle nach einem der vorhergehenden Ansprüche,
**dadurch gekennzeichnet, daß** die Lichtquelle ein Ultraviolettoder Infrarotlicht emittierender Laser ist.

6. Lichtquelle nach Anspruch 5,
**dadurch gekennzeichnet, daß** der Laser ein gepulster Erbium-, Holmium-, Thulium-, $CO_2$- oder Excimer-Laser ist.

7. Lichtquelle nach einem der vorhergehenden Ansprüche,
**dadurch gekennzeichnet, daß** die mit jedem ersten Impuls (10) bzw. Impulsabschnitt (10a) auf die Geweboberfläche aufgebrachte Bestrahlung im Bereich von 1 bis 250 $J/cm^2$ pro Impuls bzw. Impulsabschnitt liegt.

8. Lichtquelle nach einem der vorhergehenden Ansprüche,
**dadurch gekennzeichnet, daß** jeder este Impuls (10) bzw. Impulsabschnitt (10a) eine Leistung von mehr als 500 Watt und eine Dauer von 50 bis 1000 Mikrosekunden aufweist.

9. Lichtquelle nach einem der vorhergehenden Ansprüche,

**dadurch gekennzeichnet, daß** die Energie der weiteren Impulse (11; 12-14) bzw. des nachfolgenden Impulsabschnittes (10b) jeweils so bemessen ist, daß bei einer vorgegebenen Größe des Bestrahlungsfeldes die Abtragschwelle des Gewebes nicht erreicht wird.

10. Lichtquelle nach einem der vorhergehenden Ansprüche 2-9,
**dadurch gekennzeichnet, daß** der mindestens eine weitere Impuls (11) eine mit der Zeit abnehmende Bestrahlungsstärke aufweist.

11. Lichtquelle nach einem der Ansprüche 1 und 3 bis 9,
**dadurch gekennzeichnet, daß** der nachfolgende Impulsabschnitt (10b) zunächst eine mit der Zeit zunehmende und dann anschliessend abnehmende Bestrahlungsstärke aufweist.

12. Lichtquelle nach einem der Ansprüche 2 bis 10,
**dadurch gekennzeichnet, daß** die einzelnen aufeinanderfolgenden weiteren Impulse (12-14) einer Impulsfolge jeweils eine abnehmende Bestrahlungstärke, jedoch eine zunehmende Impulsdauer aufweisen und daß der Energieinhalt eines jeden dieser Impulse bei einer vorgegebenen Größe des Bestrahlungsfeldes unter dem Abtragschwellenwert liegt.

13. Lichtquelle nach einem der Ansprüche 2 bis 10 und 12,
**dadurch gekennzeichnet, daß** jede Impulsfolge eine vorgegebene Dauer aufweist.

14. Lichtquelle nach einem der vorhergehenden Ansprüche,
**dadurch gekennzeichnet, daß** der Energieinhalt des auf den ersten Impuls (10) bzw. ersten Impulsabschnitt (10a) folgenden Impulses (11) bzw. der folgenden Impulsserien (12-14) bzw. des nachfolgenden Impulsabschnittes (10b) für eine Koagulation des Gewebes ausreichend ist.

**Claims**

1. Pulsed light source for removing biological tissue, with a control unit for controlling the light source in such a way that it delivers a sequence of pulses, of a predetermined duration and radiation intensity in each case,
wherein the control unit can be operated in such a way that the light source delivers pulses (10) of the same wavelength with a predetermined repeat frequency,
wherein the pulses (10) in each case comprise a first pulse section (10a) of a short duration with a radiation intensity and radiation sufficient for removing tissue, and a subsequent pulse section (10b) whose radiation intensity can be controlled separately from the pulse section (10a) and is not sufficient for the removal of tissue but yields a heat effect.

2. Pulsed light source for removing biological tissue, with a control unit for controlling the light source in such a way that it delivers a sequence of pulses, of a predetermined duration and radiation intensity in each case,
wherein the control unit can be operated in such a way that the light source delivers pulses (10) of the same wavelength with a predetermined repeat frequency,
wherein the pulses (10) comprise at least one first pulse (10) with a radiation intensity and radiation sufficient for removing tissue, and at least one further pulse (11; 12-14) which has a radiation intensity that can be controlled separately from the at least one first pulse (10), and which is not sufficient for the removal of tissue but yields a heat effect.

3. Light source in accordance with one of the claims 1 or 2, **characterised in that** the at least one first pulse (10) or first pulse section (10a) results in low-damage removal of tissue with only slight coagulation of the areas at the edge of the removal.

4. Light source in accordance with one of the claims 1 to 3, **characterised in that** the at least one first pulse (10) or first pulse section (10a) has a high radiation intensity and the emitted light has high absorption in the tissue.

5. Light source in accordance with one of the preceding claims, **characterised in that** the light source is a laser that emits an ultra-violet or infra-red light.

6. Light source in accordance with claim 5, **characterised in that** the laser is a pulsed erbium, holmium, thulium, $CO_2$ or excimer laser.

7. Light source in accordance with one of the preceding claims, **characterised in that** the radiation that is applied to the surface of the tissue with each first pulse (10) or pulse section (10a) lies within the range of 1 to 250 $J/cm^2$ per pulse or pulse section.

8. Light source in accordance with one of the preceding claims, **characterised in that** each first pulse (10) or pulse section (10a) has an output of more than 500 watts and a duration of 50 to 1000 microseconds.

9. Light source in accordance with one of the preceding claims, **characterised in that** the energy of the further pulses (11; 12-14) or of the subsequent pulse section (10b) is in each case proportioned such that in the case of a given size of the radiation field, the threshold for removal of tissue is not reached.

10. Light source in accordance with one of the preceding claims 2-9, **characterised in that** the at least one further pulse (11) has a radiation intensity that decreases over time.

11. Light source in accordance with one of the claims 1 and 3 to 9, **characterised in that** the subsequent pulse section (10b) has a radiation intensity that initially increases over time and then subsequently decreases over time.

12. Light source in accordance with one of the claims 2 to 10, **characterised in that** the individual further pulses (12-14) of a pulse sequence that follow on from one another in each case have a decreasing radiation intensity, but an increasing pulse duration, and that in the case of a given size of the radiation field, the energy content of each one of these pulses lies below the threshold value for removal.

13. Light source in accordance with one of the claims 2 to 10 and 12, **characterised in that** each pulse sequence has a given duration.

14. Light source in accordance with one of the preceding claims, **characterised in that** the energy content of the pulse (11) that follows the first pulse (10) or first pulse section (10a), or of the following pulse series (12-14), or of the subsequent pulse section (10b), is sufficient for coagulation of the tissue.

**Revendications**

1. Source de lumière pulsée pour l'ablation de tissus biologiques, avec une unité de commande pour la commande de la source de lumière de telle manière que celle-ci délivre une suite d'impulsions, chacune d'une durée et d'une intensité d'irradiation données, l'unité de commande étant activable de telle manière que la source de lumière délivre des impulsions (10) de même longueur d'onde à une fréquence de répétition donnée, les impulsions (10) comprenant chacune une première partie d'impulsion (10a) de courte durée ayant une intensité d'irradiation et une irradiation suffisantes pour l'ablation de tissus biologiques et une partie d'impulsion suivante (10b) dont l'intensité d'irradiation est commandable séparément de celle de la première partie d'impulsion (10a) et n'est pas suffisante pour l'ablation de tissus biologiques mais produit néanmoins un effet thermique.

2. Source de lumière pulsée pour l'ablation de tissus biologiques, avec une unité de commande pour la commande de la source de lumière de telle manière que celle-ci délivre une suite d'impulsions, chacune d'une durée et d'une intensité d'irradiation données, l'unité de commande étant activable de telle manière que la source de lumière délivre des impulsions (10) de même longueur d'onde à une fréquence de répétition donnée, les impulsions (10) étant composées d'au moins une première impulsion (10) ayant une intensité d'irradiation et une irradiation suffisantes pour l'ablation de tissus biologiques et d'au moins une autre impulsion (11 ; 12-14) ayant une intensité d'irradiation commandable séparément de celle de la ou les première(s) impulsion(s) (10), laquelle n'est pas suffisante pour l'ablation de tissus biologiques mais produit néanmoins un effet thermique.

3. Source de lumière selon l'une des revendications 1 ou 2,
**caractérisée par le fait que** la ou les première(s) impulsion(s) (10) ou la première partie d'impulsion (10a) produisent une ablation de tissus peu dommageable avec seulement une faible coagulation des zones marginales.

4. Source de lumière selon l'une des revendications 1 à 3,
**caractérisée par le fait que** la ou les première(s) impulsion(s) (10) ou la première partie d'impulsion (10a) présentent une intensité d'irradiation élevée et que la lumière émise présente une absorption élevée dans les tissus.

5. Source de lumière selon l'une des revendications précédentes,
**caractérisée par le fait que** la source de lumière est un laser émettant de la lumière ultraviolette ou infrarouge.

6. Source de lumière selon la revendication 5,
**caractérisée par le fait que** le laser est un laser pulsé à l'erbium, à l'holmium, au thulium, au $CO_2$ ou excimère.

7. Source de lumière selon l'une des revendications précédentes,
**caractérisée par le fait que** l'irradiation appliquée avec chaque première impulsion (10) ou partie d'impulsion (10a) sur la surface des tissus se situe dans la plage comprise entre 1 et 250 $J/cm^2$ par impulsion ou partie d'impulsion.

8. Source de lumière selon l'une des revendications précédentes,
**caractérisée par le fait que** chaque première impulsion (10) ou partie d'impulsion (10a) présente une puissance de plus de 500 watts et une durée de 50 à 1000 microsecondes.

9. Source de lumière selon l'une des revendications précédentes,
   **caractérisée par le fait que** l'énergie des impulsions suivantes (11 ; 12-14) ou de la partie d'impulsion suivante (10b) est chaque fois calculée pour que, pour une grandeur donnée du champ d'irradiation, le seuil d'ablation des tissus ne soit pas atteint.

10. Source de lumière selon l'une des revendications précédentes 2 à 9,
    **caractérisée par le fait que** la ou les impulsion(s) suivante(s) (11) présentent une intensité d'irradiation décroissante avec le temps.

11. Source de lumière selon l'une des revendications précédentes 1 et 3 à 9,
    **caractérisée par le fait que** la partie d'impulsion suivante (10b) présente une intensité d'irradiation d'abord croissante avec le temps et ensuite décroissante avec le temps.

12. Source de lumière selon l'une des revendications précédentes 2 à 10,
    **caractérisée par le fait que** les différentes impulsions suivantes (12-14) successives d'une suite d'impulsions présentent chacune une intensité d'irradiation décroissante mais une durée d'impulsion croissante et que le contenu énergétique de chacune de ces impulsions se situe sous le seuil d'ablation pour une grandeur donnée du champ d'irradiation.

13. Source de lumière selon l'une des revendications précédentes 2 à 10 et 12,
    **caractérisée par le fait que** chaque suite d'impulsions présente une durée donnée.

14. Source de lumière selon l'une des revendications précédentes 2 à 10 et 12,
    **caractérisée par le fait que** le contenu énergétique de l'impulsion suivante (11) ou des séries d'impulsions suivantes (12-14) ou de la partie d'impulsion suivante (10b) succédant à la première impulsion (10) ou à la première partie d'impulsion (10a) est suffisant pour une coagulation des tissus.

Krater

Karbonisationszone

Vakuolenzone

Koagulationszone

Zone reversibler Schäden

Fig. 1

Krater

Koagulationszone

Zone reversibler Schäden

Fig. 2

Krater

Koagulationszone

Zone reversibler Schäden

Fig. 3

Fig. 4

Fig. 5

Fig. 6

Wärmestrahlung

F

D

V

S

Laserstrahlung

Q

L

A

Gewebe

Fig. 7